# EUROPEAN PATENT APPLICATION

(11) **EP 1 621 081 A1**
(43) Date of publication of application: **01.02.2006**
(21) Application number: 04730741.8
(22) Date of filing: 30.04.2004
(51) Int. Cl.: A23L 1/052, A23G 1/00, A23G 3/00

(54) **MATERIAL FOR INHIBITING ENAMEL DECALCIFICATION**

(30) Priority: 30.04.2003 JP 2003124725
(71) Applicant: Asahi Breweries, Ltd., Tokyo 104-0031 (JP)
(72) Inventor: IMAI, Susumu, Tokyo 1890022 (JP); TAGASHIRA, Motoyuki, c/o Asahi Breweries, Ltd., Moriya-shi, Ibaraki 3020106 (JP); KANDA, Tomomasa, c/o Asahi Breweries, Ltd., Moriya-shi, Ibaraki 3020106 (JP); NISHIZAWA, Toshiki, Tokyo 1760022 (JP); HANADA, Nobuhiro, Tokyo 1690072 (JP)
(74) Representative: Ziebig, Marlene
(86) International application number: PCT/JP2004/006465
(87) International publication number: WO 2004/096165

(57) **Abstract**

It is intended to provide an effective cariostatic material which inhibits dental plaque formation as well as onset of dental carries. A material for inhibiting enamel demineralization containing, as the active ingredient, a proanthocyanidin-like polyphenol originating in hop bract or immature apple, which effectively inhibits not only dental plaque formation but also the dental caries process including proliferation of bacteria, formation of acids by the bacteria and enamel demineralization. Also, foods, drinks and oral care goods with the use of the above substance as an enamel demineralization inhibitor are provided.

## Description

### Technical Field

The present invention provides a material for inhibiting enamel demineralization and the use. The material contains, as the active ingredient, a proanthocyanidin-like polyphenol, particularly originating immature apples or hop bract.

### Background Art

According to actual investigation of dental diseases in 1999 through Ministry of Health, Labour and Welfare, 80 % of Japanese people having permanent teeth have any dental caries. It is said that such dental caries are one of national diseases.

Dental carries are a disease caused by bacteria. Mutans Streptococci such as *S*. *mutans* and *S. sobrinus* produces water-insoluble grucan from sucrose, and adheres on the surface of teeth along with bacteria to form dental plaque. Then, several kinds of bacteria such as dental bacteria propagate in the dental plaque, pH of dental surface is lowered by organic acids which are produced by bacteria metabolism, dental enamel elutes to form a hole, and dental carries are produced. Namely, it is said that dental carries occur in a series of steps, formation of dental plaque, proliferation of bacteria, formation of acids by the bacteria and enamel demineralization.

As a method for preventing dental carries, there are some known methods such as a method for strengthening the quality of teeth with fluorine, a method for using an antibacterial material for bacteria of dental caries, a method for using an alternative sugar, which does not produce glucan, instead of sugar, and a method for using an inhibitor of an enzyme producing a water-insoluble glucan. However, these methods have merits and demerits in the effect, cost and influence for nutrition. Considering morbidity rate of dental caries do not much decrease at present, it is difficult to say that these methods are perfect.

In recent years, the total of medical cost in Japan is about 30 trillion-Yen and the dental fee amounts to 2.5 trillion. Judging from not only improvement of QOL based on promotion of health, but also inhibition and decrease of the medical cost, the development of a simple and good method for effectively preventing to get dental carries has much significance industrially.

As to effects of anti-dental carries, there are some reports concerning an extract of green tea leaves (Publications of Japanese Patent Laid-open Hei 01-9922, 01-90124 and 01-265010), an extract of Oolong tea leaves (Japanese Patent Laid-open Hei 03-284625 publication) and the like. As to polyphenol derived from immature apples or hop bract, the inventors of the present invention have reported the effect for inhibiting the formation of dental carries (Publications of Japanese Patent Laid-open Hei 07-285876 and 09-295944. These effects of anti-dental carries are based on the above publications of Japanese Patent Laid-open Hei 01-9922, 01-90124 and 01-265010, in which the formation of dental plaque depressed by the polyphenol having antibacterial effects to bacteria of dental carries is described, or in the above publications of Japanese Patent Laid-open Hei 03-284625, 07-285876 and 09-295944, in which the formation of dental plaque depressed by the polyphenol inhibiting an enzyme (GTF) producing water-insoluble glucan is described.

However, as shown in the above, the dental caries are formed by a series of steps of formation of dental plaque, proliferation of bacteria, formation of acids by the bacteria and enamel demineralization. As an example, it is difficult to remove perfectly the bacteria of dental carries by using the polyphenol having antibacterial effects. The bacteria of dental carries again proliferate in the mouth with time. Even if the formation of dental plaque is efficiently inhibited by inhibiting GTF, small amount of dental carries is advanced by the next steps to produce dental carries. Accordingly, even if the number of bacteria of dental carries is decreased or the formation of dental plaque is efficiently inhibited, it is not evidence of the inhibition of the formation of dental Prague. Accordingly, the present invention aims to provide an effective cariostatic material, which inhibits dental plaque formation as well as onset of dental carries.

### Disclosure of Invention

The present inventors have earnestly studied the above problems and have found that a proanthocyanidin-like polyphenol originating in hop bract or immature apple is a material that effectively inhibits not only dental plaque formation but also the dental carries process including proliferation of bacteria, formation of acids by the bacteria and enamel demineralization. Further, the present invention has been attained by the use of the material as a cariostatic material of drinks and oral care goods.

The polyphenol is a generical name of compounds having several phenolic hydroxyl groups in the molecules contained in plants. The proanthocyanidin-like polyphenol is a polyphenol and a compound producing anthocyanidin (cyanidin or derphynidin or properargonin) of a red pigment by hydrolysis.

Namely, the first of the present invention relates to a material inhibiting enamel demineralization, which is a proanthocyanidin-like polyphenol. The second of the present invention relates to a material inhibiting enamel demineralization which is a proanthocyanidin-like polyphenol originating in immature apple. The third of the present invention relates to a material inhibiting enamel demineralization, which is a proanthocyanidin-like polyphenol originating in hop bract.

### Brief Description of Drawings

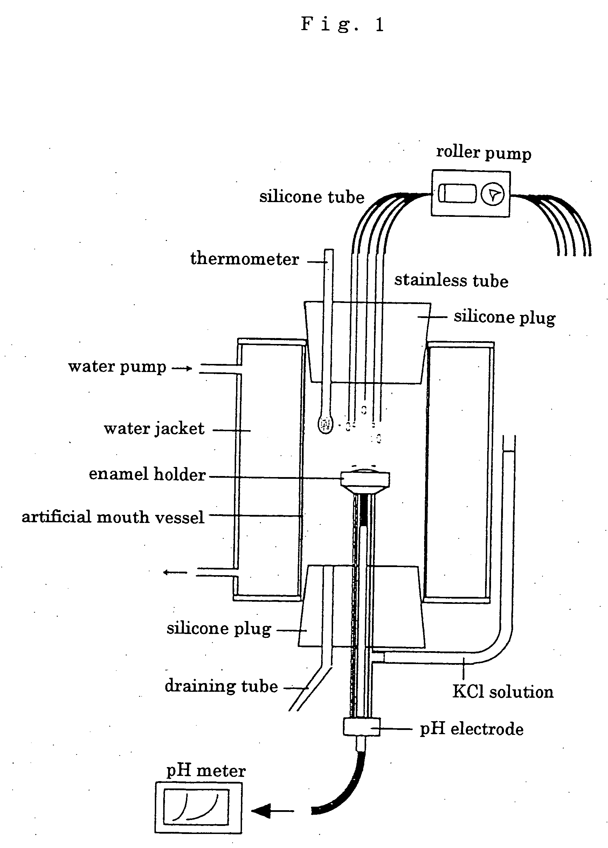

Fig. 1 shows a cross section drawing of a center part of an artificial mouth device.

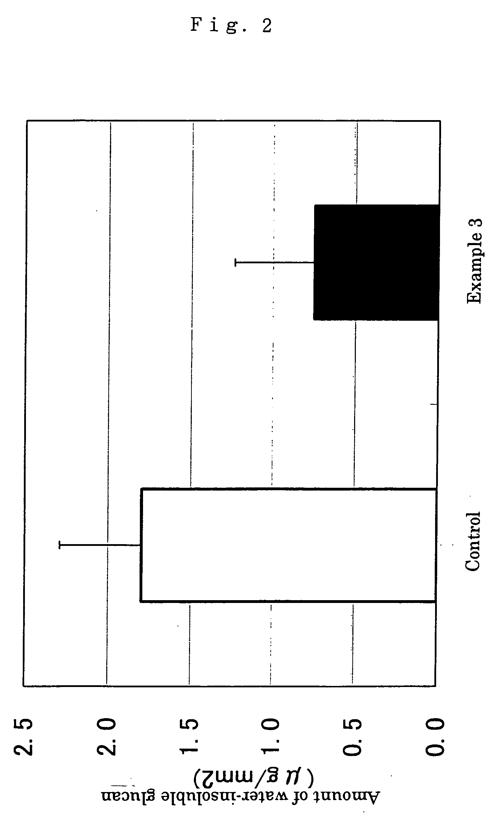

Fig. 2 shows a graph showing the compared amounts of water-insoluble glucan (corresponding to the dental plaque formed artificially) on enamel tooth pieces after the test using *S. mutans.* When the material inhibiting enamel demineralization obtained in Example 3 is added (right), the amount of water-insoluble glucan decreases more than that of control (left).

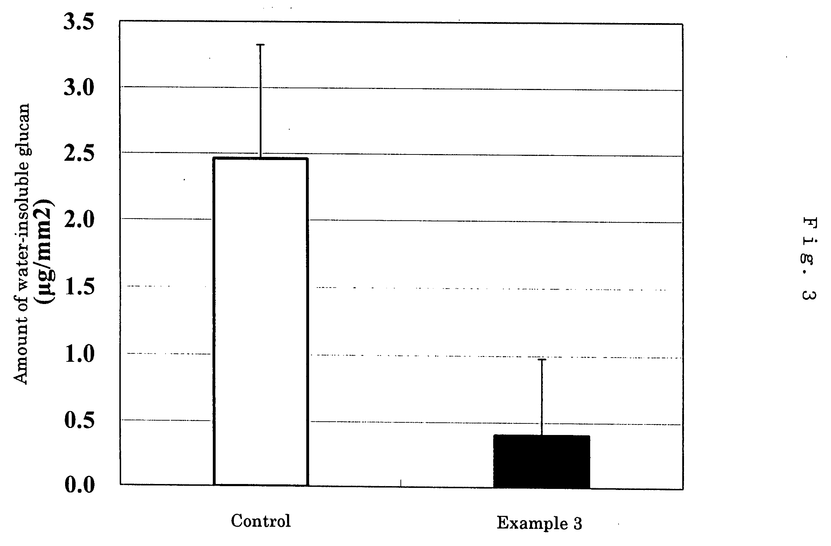

Fig. 3 shows a graph showing the compared amounts of water-insoluble glucan (corresponding to the dental plaque formed artificially) on enamel tooth pieces after the test using *S. sorbrinus.* When the material inhibiting enamel demineralization obtained in Example 3 is added (right), the amount of water-insoluble glucan decreases more than that of control (left).

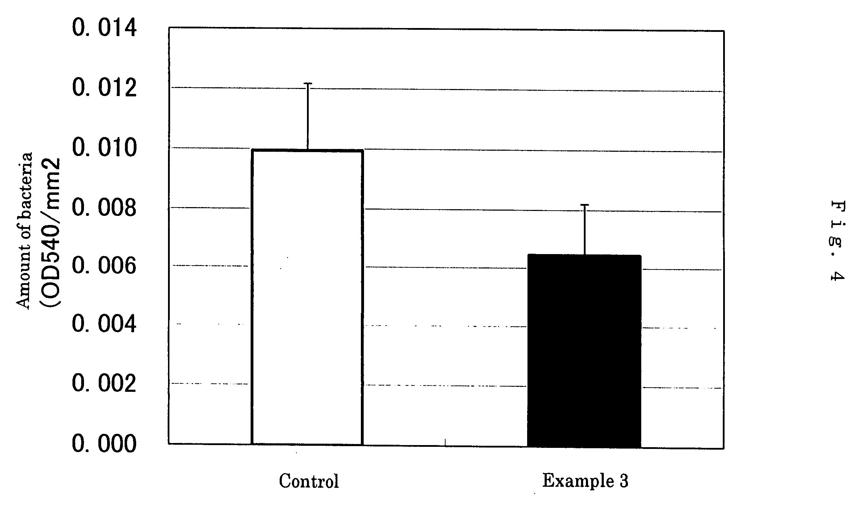

Fig. 4 shows a graph showing the compared amounts of bacteria of dental carries on enamel tooth pieces after the test using *S. mutans.* When the material inhibiting enamel demineralization obtained in Example 3 is added (right), the amount of bacteria of dental carries decreases more than that of control (left). It shows that the colonization and proliferation of the bacteria of dental carries are inhibited in the artificial dental plaque.

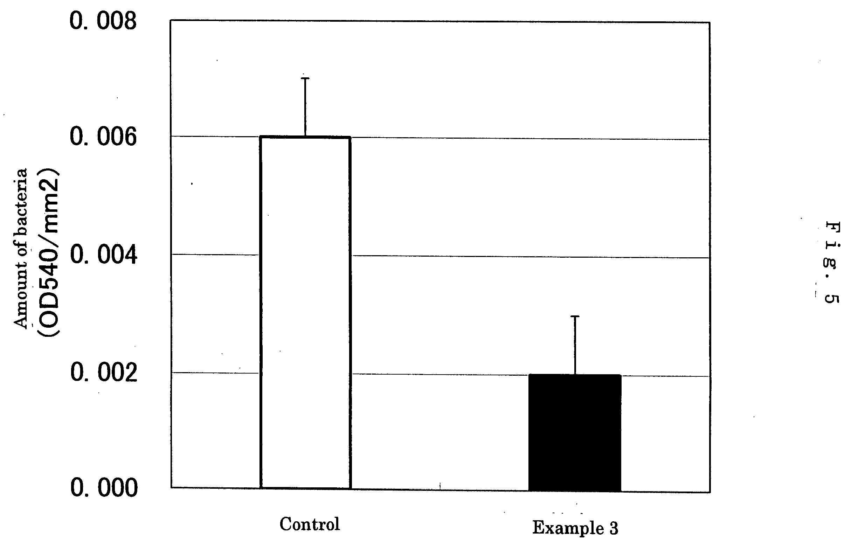

Fig. 5 shows a graph showing the compared amounts of bacteria of dental carries on enamel tooth pieces after the test using *S. sorbrinus.* When the material inhibiting enamel demineralization obtained in Example 3 is added (right), the amount of bacteria of dental carries decreased more than that of control (left). It shows that the colonization and proliferation of the bacteria of dental carries are inhibited in the artificial dental plaque.

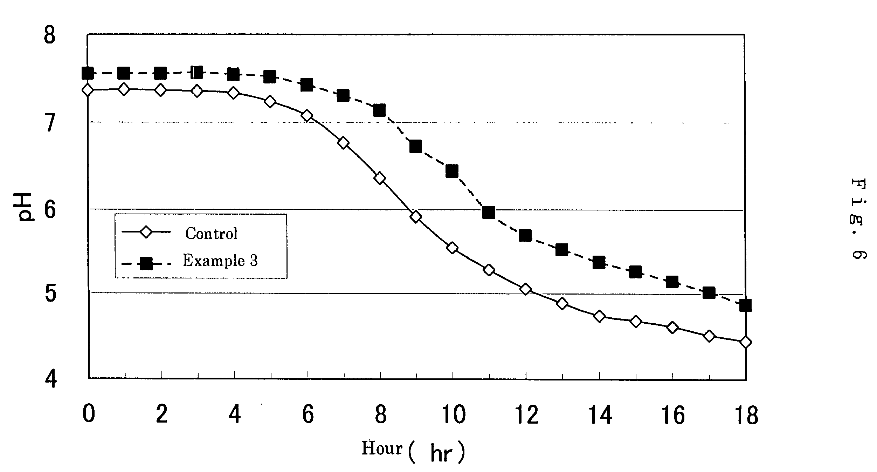

Fig. 6 shows a graph showing the pH change of the plane electrode formed directly under the artificial dental plaque in the experiment using *S. mutans.* When the material inhibiting enamel demineralization obtained in Example 3 is added (right), the change of pH decreases more than that of control (left). (pH value does not so lower). It shows that the amount of acid formation decreases in the artificial dental plaque.

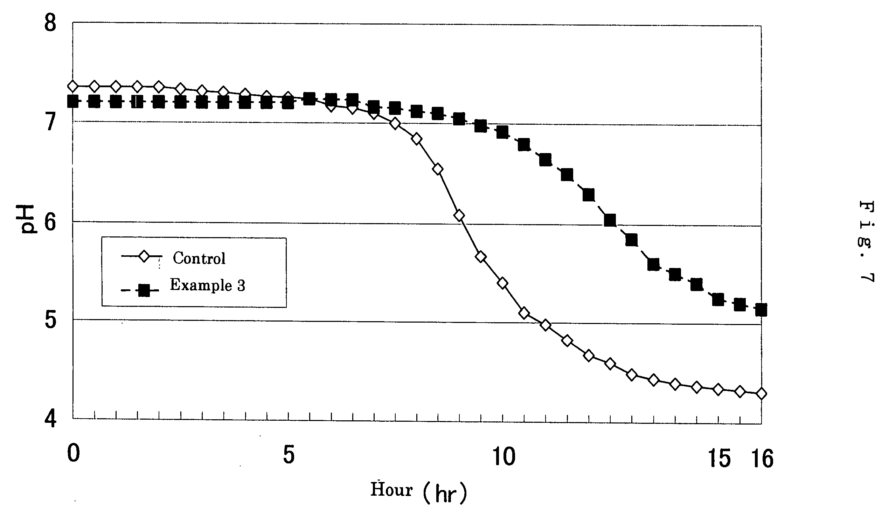

Fig. 7 shows a graph showing the pH change of the plane electrode formed directly under the artificial dental plaque in the experiment using *S. sorbrinus.* When the material inhibiting enamel demineralization obtained in Example 3 is added (right), the change of pH decreases more than that of control (left). (pH value does not so lower). It shows that the amount of acid formation decreases in the artificial dental plaque.

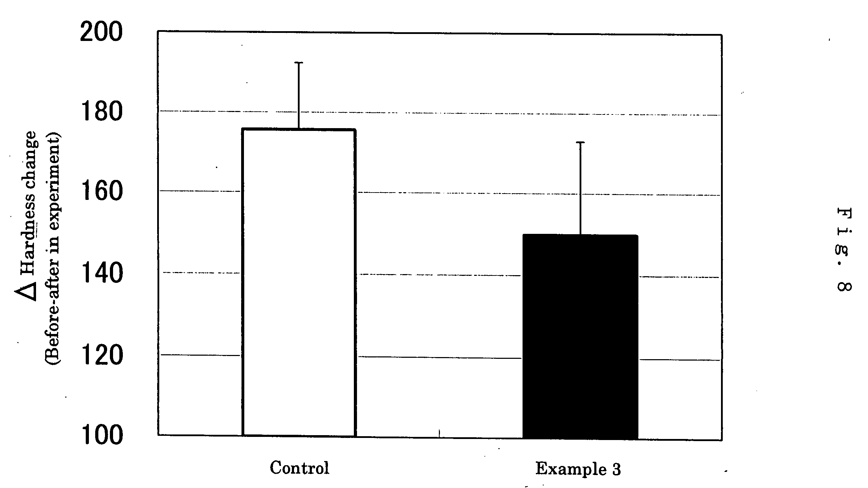

Fig. 8 shows a graph showing hardness change of enamel tooth pieces before and after in the experiment using *S. mutans.* When the material inhibiting enamel demineralization obtained in Example 3 is added (right), the hardness change of enamel tooth pieces is inhibited more than that of control (left).

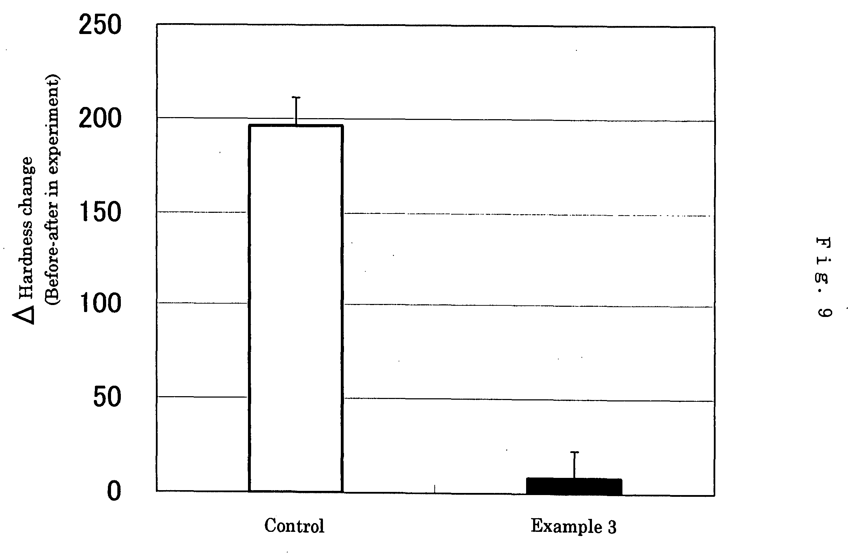

Fig. 9 shows a graph showing hardness change of enamel tooth pieces before and after in the experiment using *S. sobrinus.* When the material inhibiting enamel demineralization obtained in Example 3 is added (right), the hardness change of enamel tooth pieces is inhibited more than that of control (left).

### Best Mode for carrying Out the Invention

In the present invention, immature apple as the raw material means the superfluous fruits artificially thinned out or naturally drop down before ripening.

In the present invention, hop bract as the raw material means that formed by removing the lupulin part from the hop cone. Generally, the hop bract is obtained by removing the lupulin part after crushing and shieving the hop cone. However, in recent beer brewing, in order to omit the step of removing the hop bract by sieving, the hop bract is formed into a pellet without removing the hop bract unnecessary in beer brewing. Such obtained hop pellet tends to be used in beer brewing. Accordingly, as the raw material in the present invention, the material containing the hop bract can be used without limit, and further the hop cone and hop pellets containing the hop bract can be used as the raw material without problems.

The raw material is compressed to squeeze the juice out, or extracted with an aqueous alcohol solution to obtain a solution containing the material for inhibiting enamel demineralization, and it may be used as powder. If necessary, the material for inhibiting enamel demineralization may be used by purifying with a column filled with granular resin having affinity for polyphenol so as to more purify.

The resulting material for inhibiting enamel demineralization may be used for confectionary, food and drink, such as candy, chocolate, caramels, chewing gum and the like staying in the mouth at a relative long time. The material also may be used by adding to buccal preparations such as gargle liquid and dentifrice. When the cariostatic material is added to these food and drink, and buccal preparations, the cariostatic material may be used by adding as it is powder. Preferably the cariostatic material may be added to obtain an aqueous or alcohol solution of 1-2%. The final concentration is preferably 1-5000ppm, more preferably 100-2000ppm for food and drink or buccal preparations.

Although the following examples are described detailly the embodiment of the present invention, the invention is not limited into these examples.

### Example 1 (Preparation of the material for inhibiting enamel demineralization from immature apple)

Immature apples (average weight 5.03g/apple) 400g was homogenized with 1% hydrochloric acid methanol (200ml), and extracted by heating and refluxing (three times). The extract liquid was concentrated under reduced pressure to remove methanol. After adding chloroform (200ml) to the concentrate, the solution was distributed (two times). After the aqueous layer was recovered and filtered, 200ml was measured with a messcylinder. The solution was purified by a solid phase extraction method and freeze-dried to obtain yellow material for inhibiting enamel demineralization 0.5g.

### Example 2 (Preparation of the material for inhibiting enamel demineralization from hop bract)

Hop bract 50g was extracted with 1000ml of an aqueous solution of 40% ethanol by stirring at a temperature of 50°C for 60 minutes. After filtration, the solution was concentrated under reduced pressure to obtain 500ml. The concentrate was passed through a column filled with styrene-divinyl benzene resin (manufactured by Mitsubishi Kagaku Co., Sepabeads 70) 150ml and washed with water 500ml. Further, 600ml of an aqueous solution of 50% ethanol was passed through the column. The eluted liquid was recovered, and freeze-dried to obtain the material for inhibiting enamel demineralization as orderless pale yellow powder having faint bitter taste 1.7g. The yield from the hop bract was 3.4%.

### Example 3 (Preparation of the material for inhibiting enamel demineralization with a ultrafilter film)

The material for inhibiting enamel demineralization 5g obtained by using the same method described in Example 2 was dissolved in 500ml of an aqueous solution of 50 % ethanol and treated with a ultrafilter film having fraction molecular weight 10,000. The upper residue liquid not passed through the film was concentrated and freeze-dried, and further purified material for inhibiting enamel demineralization 2.2g was obtained as yellow to brown powder.

**Example 4 (Dentifrice)**

| | |
|---|---|
| Dibasic calcium phosphate | 42.0 |
| Glycerin | 18.0 |
| Carrageenan | 0.7 |
| Sodium lauryl sulfate | 1.29 |
| Butyl p-hydroxybenzoate | 0.005 |
| The material obtained as in Example 1 | 0.005 |
| Flavor | 1.0 |
| Water | 37.0 |
| Total | 100.0 |

Using each component having the above weight, dentifrice was prepared by a common method. Further dentifrices were obtained by adding the materials obtained as in Examples 2 and 3 instead of the material obtained as in Example 1.

**Example 5 (Gargle)**

| | |
|---|---|
| Glycerin | 7.0 |
| Sorbitol | 5.0 |
| Ethyl alcohol | 15.0 |
| Sodium lauryl sulfate | 0.9 |
| 1-menthol | 0.05 |
| Flavor | 0.045 |
| the material obtained as in Example 1 | 0.005 |
| Water | 72.0 |
| Total | 100.0 |

Using each component having the above weight, gargle was prepared by a common method. Further, gargles were obtained by adding the materials obtained as in Examples 2 and 3 instead of the material obtained as in Example 1.

**Example 6 (Troche)**

| | |
|---|---|
| Gum arabic | 6.0 |
| Magnesium stearate | 3.0 |
| Xylitol | 73.0 |
| Maltitol | 17.6 |
| Dipotassium phosphate | 0.2 |
| Potassium phosphate | 0.1 |
| Flavor | 0.095 |
| The material obtained as in Example 1 | 0.005 |
| Total | 100.0 |

Using each component having the above weight, troche was prepared by a common method. Further, troches were obtained by adding the materials obtained as in Examples 2 and 3 instead of the material obtained as in Example 1.

**Example 7 (Wheat gluten)**

| | |
|---|---|
| Xylitol | 20.0 |
| Maltitol | 70.0 |
| Water | 9.5 |
| Coloring agent | 0.45 |
| Flavor | 0.045 |
| The material obtained as in Example 1 | 0.005 |
| Total | 100.0 |

Using each component having the above weight, wheat gluten was prepared by a common method. Further, wheat glutens were obtained by adding the materials obtained as in Examples 2 and 3 instead of the material obtained as in Example 1.

### Example 8 (Chewing gum)

| | |
|---|---|
| Gum base | 20.0 |
| Calcium carbonate | 2.0 |
| Xylitol | 77.0 |
| Stevioside | 0.095 |
| The material obtained as in Example 1 | 0.005 |
| Flavor | 0.9 |
| Total | 100.0 |

Using each component having the above weight, chewing gum was prepared by a common method. Further, chewing gums were obtained by adding the materials obtained as in Examples 2 and 3 instead of the material obtained as in Example 1.

### Example 9 (Juice)

| | |
|---|---|
| Xylitol | 20.0 |
| Citric acid | 0.2 |
| Flavor | 0.1 |
| Coloring agent | 0.15 |
| Sodium ascorbate | 0.048 |
| The material obtained as in Example 1 | 0.002 |
| Water | 79.5 |
| Total | 100.0 |

Using each component having the above weight, juice was prepared by a common method. Further, juices were obtained by adding the materials obtained as in Examples 2 and 3 instead of the material obtained as in Example 1.

### Example 10 (Cookies)

| | |
|---|---|
| Soft flour | 32.0 |
| Whole egg | 16.0 |
| Butter | 16.0 |
| Maltitol | 25.0 |
| Water | 10.8 |
| Baking powder | 0.198 |
| The material obtained as in Example 1 | 0.002 |
| Total | 100.0 |

Using each component having the above weight, cookies were prepared by a common method. Further, cookies were obtained by adding the materials obtained as in Examples 2 and 3 instead of the material obtained as in Example 1.

### Example 11 (Caramels)

| | |
|---|---|
| Xylitol | 31.0 |
| Maltitol | 20.0 |
| Powder milk | 40.0 |
| Hardened oil | 5.0 |
| Sodium chloride | 0.6 |
| Flavor | 0.025 |
| The material obtained as in Example 1 | 0.005 |
| Water | 3.37 |
| Total | 100.0 |

Using each component having the above weight, caramels were prepared by a common method. Further, caramels were obtained by adding the materials obtained as in Examples 2 and 3 instead of the material obtained as in Example 1.

### Example 12 (Evaluation test for a series of steps of formation of dental caries by using an artificial mouth device)

By using an artificial mouth device (M. Hinoide, et al., Jpn. J. Oral Biol., 26, 288-291 (1984) with cow teeth pieces and typical bacteria of dental carries, *S, mutans* and *S. sorbrinus,* dental plaque formation, proliferation of bacteria, pH lowering by acid formation and enamel demineralization were studied about the effects of the material obtained in Example 3.

The outline of the artificial mouth device is shown in Fig.1. In this device, 4 pieces of cow enamel slices (3.5 × 3.5 × 1.5mm) are adhered around an electrode, and the following liquid was added dropwise from five tubes A-E placed at the upper side of the device to the pieces. By using the device, the formation of dental carries in the mouth can be reproduced.

### Materials added dropwise

Tubes A and C: HI+2.5% sucrose (1% sucrose on the electrode) (cooling)
Tubes B and D: Electrode 1; PBS (cooling)
Electrode 2; 0.125%HBT in PBS (0.05% HBT on the electrode) (when tube E is S.mutans ATCC25175)
or 0.05%HBT in PBS (0.02% HBT on the electrode) (when tube E is S.sobrinus 6715)
Tube E: PBS suspension of *S.mutans* ATCC 25175 or *S.sorbinus* 6715 (stirred on cooling).
Flow rate: 6ml/hr/tube
Temperature: 37°C (the water temperature of the water jacket was 37.5°C).

As to the evaluation, bio-film formed on the enamel slices was recovered to determine the weight of non water-soluble glucan and the amount of bacteria of dental carries.
Further, the conditions of pH lowering on the electrode plane and hardness change of the enamel slices recovered were evaluated. In addition, the weight of non water-soluble glucan was determined by a phenolic sulfuric acid method and the amount of bacteria of the dental carries was determined by the turbidity at 540nm, and the hardness of the enamel pieces was determined by using a hardness meter (manufactured by Akashi Co., MS-5 type).
The results are shown in Figs. 2-9. By adding the material obtained in Example 3, about any bacteria to be studied, as compared with control, the weight of non water-soluble glucan on the enamel pieces (correspondent to the weight of dental plaque, Figs. 2 and 3) and the amount of bacteria of the dental carries (Figs. 4 and 5) were decreased, and the pH lowering on the pieces was depressed (Figs. 6 and 7). Moreover, the enamel hardness little lowered comparing with remarkable increase of the control (Figs. 8 and 9). It appears that the enamel demineralization was markedly inhibited. Namely, by adding the material obtained in Example 3, it appears that all formation steps of the dental caries including proliferation of bacteria, formation of acids by the bacteria and enamel demineralization were efficiently inhibited.

## Claims

1. A material for inhibiting enamel demineralization containing as a principal component a proanthocyanidin-like polyphenol.

2. A material for inhibiting enamel demineralization as claimed in claim 1, the proanthocyanidin-like polyphenol originates in immature apple.

3. A material for inhibiting enamel demineralization as claimed in claim 1, the proanthocyanidin-like polyphenol originates in hop bract.

4. An eating and drinking product containing the material for inhibiting enamel demineralization as claimed in claim 1.

5. An eating and drinking product containing the material for inhibiting enamel demineralization as claimed in claim 2.

6. An eating and drinking product containing the material for inhibiting enamel demineralization as claimed in claim 3.

7. An oral care good containing the material for inhibiting enamel demineralization as claimed in claim 1.

8. An oral care good containing the material for inhibiting enamel demineralization as claimed in claim 2.

9. An oral care good containing the material for inhibiting enamel demineralization as claimed in claim 3.
